(19) Europäisches Patentamt
European Patent Office
Office européen des brevets

(11) Numéro de publication: **0 135 406**
**B1**

(12) **FASCICULE DE BREVET EUROPEEN**

(45) Date de publication du fascicule du brevet: **07.10.87**

(51) Int. Cl.⁴: **C 07 D 207/273**, C 07 D 407/06, C 07 D 409/06, A 61 K 31/40

(21) Numéro de dépôt: **84401419.1**

(22) Date de dépôt: **04.07.84**

(54) **Pyrrolidinones actives sur le système nerveux central, leur procédé de préparation, les médicaments en contenant.**

(30) Priorité: **12.07.83 FR 8311636**

(43) Date de publication de la demande: **27.03.85 Bulletin 85/13**

(45) Mention de la délivrance du brevet: **07.10.87 Bulletin 87/41**

(84) Etats contractants désignés: **AT BE CH DE FR GB IT LI LU NL SE**

(56) Documents cités: **EP - A - 0 010 460**

(73) Titulaire: **SANOFI, 40, Avenue George V, F-75008 Paris (FR)**

(72) Inventeur: **Bizière, Kathleen, 6, Lotissement Rayons d'Oc, F-34170 Clapiers (FR)**
Inventeur: **Chambon, Jean-Pierre, Chemin des Truquets Route de Montpellier, F-34570 Montarnaud (FR)**
Inventeur: **Molimard, Jean-Charles, 782 Rue des Combelles, F-34980 Saint-Gely du Fesc (FR)**

(74) Mandataire: **Gillard, Marie-Louise et al, Cabinet Beau de Loménie 55, Rue d'Amsterdam, F-75008 Paris (FR)**

EP 0 135 406 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne les pyrrolidinones de formule générale (I):

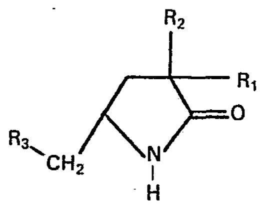

(I)

dans laquelle:

– $R_1$ représente un atome d'hydrogène ou un alkyle

– $R_2$ représente un alkyle, un phényle ou un triméthylsilyle

– $R_3$ représente un thiényl-2 ou un phényle éventuellement substitué 1 ou 2 fois par un halogène, un méthyle, un méthoxy ou un groupe trifluorométhyle.

Tout au long de la présente description le terme alkyle représente un alkyle droit ou ramifié comprenant 1 à 4 atomes de carbone.

Ces composés présentent une activité sur le système nerveux central; ils ont en particulier un effet anticonvulsivant sur l'animal et agissent sur la motilité spontanée.

Les composés selon l'invention peuvent être préparés suivant plusieurs méthodes de synthèse selon la valeur des substituants $R_1$, $R_2$, $R_3$. La préparation s'effectue soit par cyclisation d'un ester γ-aminé, soit par substitution sur un noyau pyrrolidine, ces deux méthodes peuvent se combiner.

Procédé 1:

Les composés selon l'invention peuvent être obtenus par cyclisation à chaud d'un ester γ-aminé:

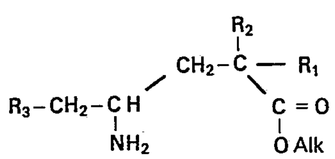

(II)

La préparation de (II) peut s'effectuer par différentes méthodes.

Lorsque $R_3$ est différent du thiényle, on peut opérer selon le schéma réactionnel suivant:

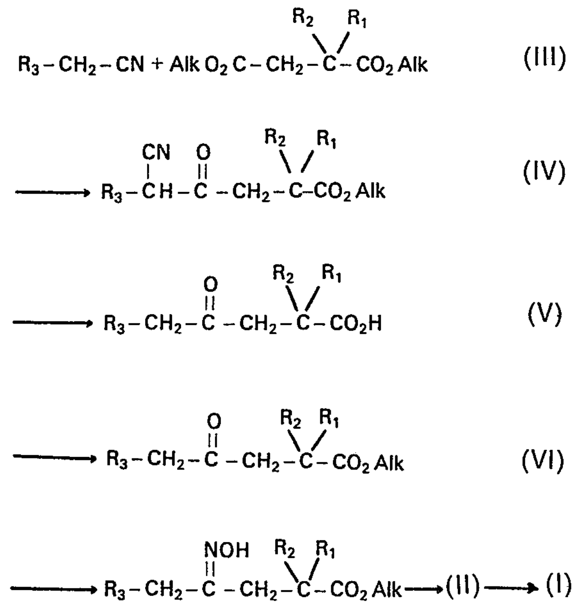

(III)

(IV)

(V)

(VI)

(VII)

Après avoir condensé le cyanure de benzyle (éventuellement substitué) sur le diester succinique substitué, en présence d'éthylate de sodium, on hydrolyse en milieu acide pour obtenir le γ-céto-acide (V) que l'on estérifie ensuite de la manière habituelle.

Le γ-céto-ester (VI) est transformé en γ-amino-ester (II), soit par une amination réductrice par l'acétate d'ammonium en présence de cyanoborohydrure de sodium, soit par la formation d'un γ-hydroxyiminoester que l'on soumet à une hydrogénation catalytique.

Pour préparer les pyrrolidinones selon l'invention qui portent un substituant alkyle en position 3, on peut préparer (II) selon le schéma réactionnel suivant:

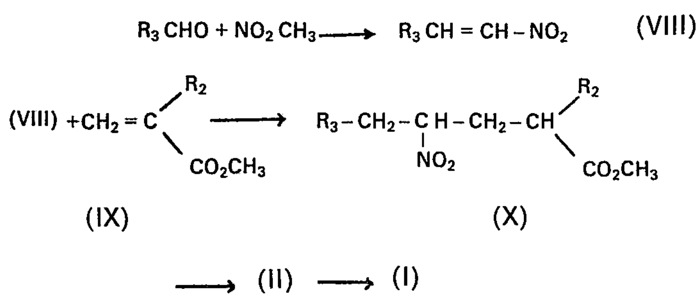

(VIII)

(IX)

(X)

La condensation d'un nitroalcane sur un aldéhyde pour donner un nitrostyrène est décrite dans: J. Org. Chem, 1953, 18, p. 1, pour les benzaldéhydes et dans: J. Org. Chem., 1949, 14, p. 409, pour les thiophènealdéhydes.

L'addition, sur le nitrostyrène (VIII) d'un méthacrylate (IX), s'effectue à température ambiante en présence de borohydrure de sodium dans un solvant, tel que le diméthylsulfoxyde.

Par hydrogénation catalytique de (X), sous une pression de quelques atmosphères, on obtient le γ-amino-ester que l'on cyclise à chaud.

Procédé 2:

Pour préparer les pyrrolidinones selon l'invention qui sont mono- ou di-substitués en position 3 par un groupement alkyle ou triméthylsilyle, on peut également utiliser le procédé 2:

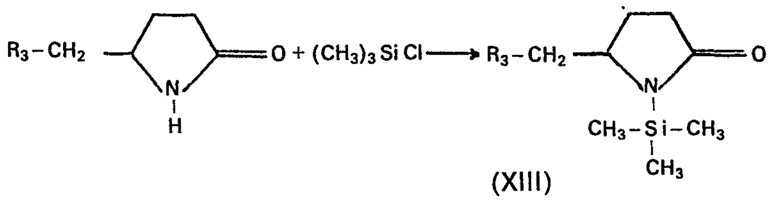

(XIII)

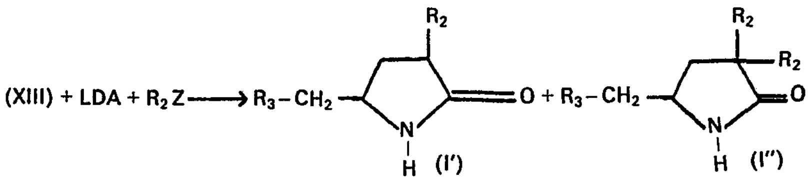

(I') (I'')

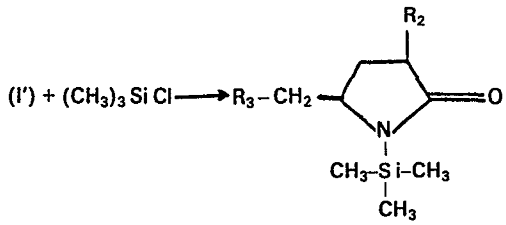

(XIV)

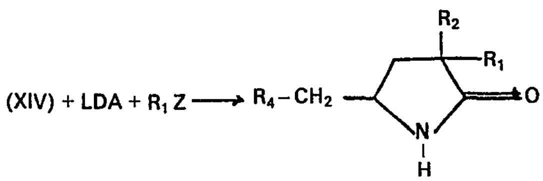

La substitution de la pyrrolidinone par $R_1$ et $R_2$ nécessite la protection du groupement NH par le groupement triméthylsilyle. Par action du lithium diisopropylamide (LDA), on forme un anion sur lequel on fait réagir un halogénure $R_1$–Z [Z=Br ou I si $R_1$=alkyle et Z =Cl si $R_1$ = Si $(CH_3)_3$]. On obtient, à côté du produit monosubstitué en position 3 par $R_1$, un produit disubstitué en position 3 par deux radicaux $R_1$. A partir du produit monosubstitué et d'un halogénure $R_2$–Z, la même séquence de réaction conduit au produit disubstitué par les radicaux $R_1$ et $R_2$.

Exemple 1:

Ethyl-3[(fluoro-2-phényl)méthyl]-5-méthyl-3-pyrrolidinone-2: SR 42001.

a) – Ester éthylique de l'acide céto-4 éthyl-2 (fluoro-2 phényl)-5 méthyl-2 pentanoïque.

On dissout 18,5 g de sodium dans 250 ml d'alcool éthylique absolu, puis on ajoute 110 g du diester diméthylique de l'acide éthyl-2 méthyl-2 succinique et 72 g de fluoro-2- cyanure de benzyle.

On porte à reflux 6 heures sous agitation puis on maintient l'agitation une nuit à température ambiante.

Après évaporation du solvant, on reprend par 500 ml d'eau puis on extrait 2 fois par 500 ml d'éther. La phase aqueuse est acidifiée à pH 4 par de l'acide acétique, puis on extrait au chloroforme, sèche sur sulfate de sodium et évapore le solvant sous vide. On ajoute au résidu 235 ml d'eau, 775 ml d'acide acétique et 260 ml d'acide chlorhydrique concentré, puis on chauffe à reflux pendant 11 heures sous azote; la solution est ensuite évaporée sous vide, reprise par du dichlorométhane, lavée à l'eau; les phases organiques sont séchées sur sulfate de sodium et concentrées sous vide.

Le résidu est repris par 300 ml d'éthanol absolu, on ajoute 6 ml d'acide sulfurique concentré puis on chauffe à reflux pendant 24 heures. Après évaporation de l'éthanol, on reprend par du dichlorométhane, lave à l'eau, par une solution de bicarbonate de sodium saturée, puis par une solution de chlorure de sodium saturée et on sèche sur sulfate de sodium.

On obtient 63 g d'ester éthylique de l'acide céto- 4 éthyl-2 (fluoro-2 phényl)-5 méthyl-2 pentanoïque.

b) – Ester éthylique de l'acide éthyl-2 (fluoro-2 phényl)-5 méthyl-2 hydroxyimino-4 pentanoïque.

63 g du γ-céto-ester préparé ci-dessus sont dissous dans 360 ml d'alcool éthylique à 96% (en volume) et 125 ml d'eau. On ajoute 19 g de chlorhydrate d'hydroxylamine et 35 g d'acétate de

sodium hydraté. On chauffe à reflux pendant 11 heures. Après évaporation du solvant, on reprend par du dichlorométhane, lave à l'eau, par une solution de chlorure de sodium saturée puis on sèche sur sulfate de sodium. Le solvant est évaporé sous vide puis le résidu est chromatographié sur 600 g silice par un mélange acétate d'éthyl-pentane (25/75 en volume).

On obtient 20 g d'ester ethylique de l'acide éthyl-2 (fluoro-2 phényl)-5 méthyl-2 hydroxyimino-4 pentanoïque.

c) – SR 42001.

20 g de l'oxime ci-dessus sont placés dans un autoclave, on ajoute 100 ml d'acide acétique à 90% et 10 g de platine sur carbone à 5%.

L'hydrogénation s'effectue sous 20 atmosphères, à température ordinaire pendant 10 heures. Après filtration sur Hyflocell, on évapore le solvant; le résidu est repris par de l'eau, on ajoute de la soude concentrée jusqu'à pH 11 et sans refroidir. Au bout de 15 minutes, on extrait par du dichlorométhane, lave à l'eau, puis par une solution de chlorure de sodium saturée, on sèche sur sulfate de sodium puis évapore sous vide. Le résidu est chromatographié sur 250 g de silice, l'éluant étant un mélange de dichlorométhane-acétate d'éthyle (60–40 en volume).

Le produit obtenue est distillé, Eb= 175–180 °C, sous 0,01 mm Hg; on obtient 1,5 g de pyrrolidinone: SR 42001.

Exemple 2:

[(fluoro-4 phényl)-méthyl]-5 méthyl-3 pyrrolidinone-2 : SR 41882.

a) – Ester éthylique de l'acide céto-4 (fluoro-4 phényl)-5 méthyl-2 pentanoïque.

On opère comme dans l'exemple 1 et avec les mêmes quantités en faisant réagir le p-fluorocyanure de benzyle avec le diester méthylique de l'acide méthyl-2 succinique.

Le γ-céto-ester obtenu est distillé sous vide; EB = 120–130 °C sous 0,05 mm Hg. On récupère 42 g de produit. b) – SR 41882.

42 g du produit précédemment obtenu sont dissous dans 800 ml de méthanol, on ajoute 135 g d'acétate d'ammonium puis 7,8 g de cyanoborohydrure de sodium. Après 16 heures d'agitation, on évapore les 3/4 de la solution sous vide puis on reprend par 400 ml d'eau, on ajoute 200 ml d'acide acétique puis on agite 1 heure avant de neutraliser par du carbonate acide de sodium. Le pH est ensuite amené à 11 par de la soude à 30%, la température monte à 50 °C.

Après 15 minutes d'agitation, on extrait par le dichlorométhane, lave, sèche sur sulfate de sodium et évapore sous vide puis sèche à nouveau 1 heure à 90 °C sous vide. Les 30 g obtenus sont chromatographiés sur 700 g de silice en utilisant l'acétate d'éthyle pur comme éluant.

Après empâtage par l'éther isopropylique, on obtient 13 g de SR 41882; Fk = 102 °C.

Exemple 3:

Diméthyl-3,3 [(fluoro-4 phényl)-méthyl]-5 pyrrolidinone-2: SR 41293.

Ce produit est préparé en suivant le même procédé que pour l'exemple 2. Après recristallisation dans le cyclohexane, Fk= 114 °C.

Exemple 4:

Méthyl-3 (thiényl-2 méthyl)-5 pyrrolidinone-2, isomère trans (SR 42008); isomère cis (SR 42009).

a) – Ester méthylique de l'acide méthyl-2- nitro-4 thiényl-5 pentanoïque.

A une solution de 23 g de nitrovinyl-2 tiophène dans 200 ml de diméthylsulfoxyde, on ajoute 21 g de méthacrylate de méthyle. En maintenant, sous agitation, la température entre 25 °C et 30 °C, on ajoute, par portions, 2,82 g de borohydrure de sodium en poudre.

Après 14 heures d'agitation, on ajoute 15 ml d'eau et 30 ml d'acide acétique, on verse dans 1 litre d'eau glacée puis on agite 30 minutes, on extrait 5 fois par 300 ml d'éther puis on lave 3 fois par 400 ml d'eau, 1 fois par 400 ml d'une solution saturée de chlorure de sodium, on sèche sur sulfate de sodium puis on évapore l'éther sous vide. Le résidu est chromatographié sur 800 g de silice, en utilisant comme éluant un mélange pentane-acétate d'éthyle (80/20 en volume).

On obtient les 2 diastéréoisomères du nitroester.

b) – SR 42008.

4,5 g du nitroester le moins polaire sont hydrogénés dans 500 ml d'éthanol à 95% avec du Nikkel de Raney T1 (1 cuillère à café) sous 10 atmosphères pendant 24 heures.

On filtre sur Hyflocell, évapore le solvant puis recristallise dans le cyclohexane. On obtient 1,5 g de l'isomère trans du produit attendu: SR 42008; Fk = 97 °C.

c) – SR 42009.

4,2 g du nitroester le plus polaire sont hydrogénés dans les mêmes conditions pour obtenir 1,6 g de l'isomère cis: SR 42009; Fk: 117 °C, après recristallisation dans le cyclohexane. L'identification de l'isomère cis se fait en résonance magnétique nucléaire par l'effet Overhauser nucléaire.

Exemple 5:

Butyl-3 [(méthoxy-3-phényl) méthyl]-5. pyrrolidinone-2: SR 42223 et Dibutyl-3,3 [(méthoxy-3-phényl) méthyl]-5- pyrrolidionone-2: SR 42224.

a) – [(méthoxy-3 phényl) méthyl)-5 méthyl-5 triméthylsilyl-1 pyrrolidinone-2.

On prépare une solution de 5 g de [(méthoxy-3 phényl) méthyl]-5 pyrrolidinone-2 dans 60 ml de benzène à laquelle on ajoute 2,5 g de triéthylamine puis, goutte à goutte, 3 g de chlorure de triméthylsilyle. On chauffe à reflux pendant 2 heures et demie puis on refroidit, filtre et évapore le benzène sous vide. 2,8 g du produit attendu distillent à 170–180 °C sous 0,1 mm de Hg.

b) – SR 42223 et SR 42224.

On dissout 1,6 g de diisopropylamine dans 50 ml de tétrahydrofuranne sec, à −40 °C, on ajoute 8,53 ml de butyllithium (1,6 M dans l'hexane) et

on maintient l'agitation à cette température sous azote pendant 20 minutes.

On refroidit à −70 °C et on ajoute sous agitation 3,2 g de triméthylsilylpyrrolidone obtenue en a) dans 20 ml de tétrahydrofuranne, on ajoute après 30 minutes 1,8 g de bromobutane; 30 minutes après, on laisse revenir à la température ambiante puis on place sous azote pendant une nuit. On ajoute ensuite une solution de chlorure d'ammonium et, après 30 minutes d'agitation, on extrait au chloroforme, lave, sèche et évapore.

Le produit est chromatographié sur 180 g de silice, en utilisant comme éluant un mélange acétate d'éthyl-dichlorométhane (20–80 en volume). Le produit disubstitué (SR 42224) est élué le premier, on en obtient 0,35 g, puis 1 g du produit monosubstitué (SR 42223), (mélange des isomères cis et trans). Ces produits sont sous forme huileuse, ils sont identifiés par leur spectre de résonance magnétique nucléaire. Le spectre est enregistré dans le chloroforme deutéré à 60 MHz, en utilisant comme référence l'hexaméthyldisiloxane.

On utilisera les abréviations suivantes:
S: singulet
Se: singulet élargi
D: doublet
T: triplet
M: multiplet
J: constante de couplage

SR 42223:

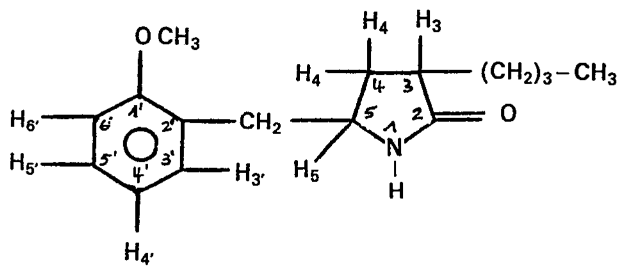

1 H à 7,30 ppm (T de D, J1 = 8 Hz, J2 = 2Hz, $H_{5'}$)
3H à 6,80 ppm (M, $H_{3'}$, $H_{4'}$, $H_5$)
1 H à 5,95 ppm (Se, CONH)
1 H à 3,80 ppm (M, $H_5$)
3H à 3,80 ppm (S, O $CH_3$)
2H à 2,70 ppm (M, $CH_2$-aromatique)
9H entre 2 et 1,5 ppm (M, $H_3$, $H_4$, $(CH_2)_3$)
3H à 0.82 ppm (T déformé, $CH_3$)

SR 42224:

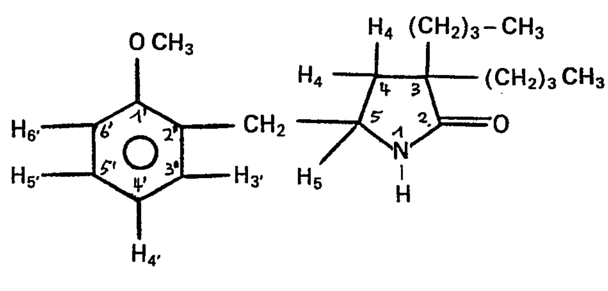

1 H à 7,23 ppm (T de D, J1 = 8 Hz, J2 = 2 Hz, $H_5$),
3H à 6,80 ppm (M, $H_3$, $H_4$, $H_6$,
1 H à 5,80 ppm (Se, CONH)
3H à 3,73 ppm (S, $OCH_3$)
1 H à 3,70 ppm (M, $H_5$)
2H à 2,70 ppm (M, $CH_2$-aromatique)
14H entre 1 et 2,2 ppm (M, $H_4$, $(CH_2)_3$–$CH_3$)
6H à 0,82 ppm (T déformé, $CH_3$).

D'autres composés selon l'invention ont également été préparés en utilisant l'un des procédés décrits plus haut. Les valeurs des substituants $R_1$, $R_2$, $R_3$ sont données dans le tableau 1 ci-dessous, ainsi que le point d'ébullition (Eb) ou le solvant de recristallisation et le point de fusion des produits (Fk). La synthèse de chaque composé a été réalisée selon la technique décrite dans l'exemple cité entre parenthèses.

Tableau 1

| N° SR (Exemple) | $R_1$ | $R_2$ | $R_3$ | Fk °C (solvant de recristallisation) ou Eb °C (pression de distillation) |
|---|---|---|---|---|
| SR 41676 (1) | H | $CH_3$ | 2,4-dichloro-phényle | Fk= 128 (CC14) |
| SR 41677 (1) | $CH_3$ | $CH_3$ | 3,4-diméthoxy-phényle | Fk=80 (i $Pr_2$ O) |
| SR 41678 (1) | H | $CH_3$ | 2-chlorophényle | Eb=180–185 (0.01 mm Hg) |
| SR 41680 (1) | $CH_3$ | $CH_3$ | 2-fluorophényle | Fk=103 (i $Pr_2$ O) |
| SR 41773 (1) | H | phényle | 3,4-diméthoxy-phényle | Fk=109 (i $Pr_2$ O) |
| SR41873 (1) | $CH_3$ | $CH_3$ | 4-chlorophényl | Fk=135 (i $Pr_2$ O) |
| SR 41880 (2) | $CH_3$ | $CH_3$ | 2-chlorophényle | Fk=86 (i $Pr_2$ O) |
| SR 41881 (2) | H | $CH_3$ | 2-fluorophényle | FK=106 (i $Pr_2$ O) |
| SR42002 (1) | $CH_3$ | $C_2H_5$ | 4-fluorophényle | Fk=96 (i $Pr_2$ O) + cyclohexane |
| SR 42003 (1) | $CH_3$ | $CH_3$ | 4-méthylphényle | Fk=129 (i $Pr_2$ O) |
| SR 42004 (1) | H | $CH_3$ | 2-chloro, 6-fluorophényle | Fk=70 (i $Pr_2$ O) |
| SR 42010 (4) | H | $CH_3$ | phényle | Fk=92 (cyclohexane) |
| SR 42133 (5) | $(CH_2)_3CH_3$ | $Si(CH_3)_3$ | phényle | Fk=88 (pentane) |
| SR 42134 (5) | H | $Si(CH_3)_3$ | phényle | Fk=134 (i $Pr_2$ O) |
| SR 42225 (5) | H | $Si(CH_3)_3$ | 4-fluorophényle | Fk=138 (i $Pr_2$ O) |
| SR 42226 (5) | H | $(CH_2)_2CH_3$ | 4-fluorophényle | Fk=45 (i $Pr_2$ O) |
| SR 42353 (1) | $CH_3$ | $CH_3$ | phényle | Fk118 (i $Pr_2$ O) |
| SR 42354 (1) | H | $CH_3$ | 4-chlorophényl | Fk=114 (i $Pr_2$ O) |
| SR 42401 (1) | H | $CH_3$ | 3-trifluorom-éthyl phényle | Fk=110 (i $Pr_2$ O) |
| SR 42508 (1) | $CH_3$ | $CH_3$ | 2,4-dichloro-phényle | Fk=130 (i $Pr_2$ O) |
| SR 42717 (1) | H | $CH_3$ | 3-chlorophényle | Fk=94 (i $Pr_2$ O) |
| SR 42725 (1) | $CH_3$ | $CH_3$ | 3-trifluorométhyl phényle | Fk=92 (i $Pr_2$ O) |
| SR 42726 (1) | H | $CH(CH_3)_2$ | 4-chlorophényle | Fk=104 (i $Pr_2$ O) |

* i $Pr_2O$ signifie l'éther isopropylique. L'activité des produits selon l'invention sur le système nerveux central a été mesurée par différents tests de pharmacologie. Pour chacun de ces tests, on a également mesuré l'acitivité d'un produit cité dans Yakugaku Zasshi, 1960, 86 (12), p 1213–1216: la phénylméthyl-5 pyrrolidinone-2 que nous appelleron composé A.

1 – Effet des produits sur la motilite spontanée

La motilité spontanée des animaux est mesurée grâce au test d'activité développé par Boissier et Simon, Arch. Int. Pharmacocyn., 1965, 158, 212–221. L'équipement est composé de cages actimétriques, type Apelab (26×21,5×10 cm), traversées par deux rayons lumineux qui impressionnent une cellule photoélectrique. Les lots sont constitués de souris femelles Charles Rivers CD1, de poids compris entre 20 et 24 g. Les animaux sont placés individuellement dans les cages 45 minutes après l'administration orale des produits à la dose de 100 mg/kg. Chaque traversée d'un faisceau lumineux est comptabilisée par un compteur individuel. Les scores correspondant aux déplacements des animaux sont enregistrés pendant 10 minutes, et comparés aux scores réalisés par les lots d'animaux témoins, uniquement traités par le véhicule (gomme arabique 5%).

Tableau 2

| Produits à 100 mg/kg per os | Acitivité locomotrice Score %/témoins |
|---|---|
| SR 41676 | −34% * |
| SR 41678 | +45% * |
| SR 41680 | +99% ** |
| SR 41881 | +41% ** |
| SR 42008 | −52% ** |
| SR 42009 | −23% * |
| Composé A | +1,2% |

*: p < ou = 0.05
**: p < ou = 0.01

Après adiministration, à la dose orale de 100 mg/kg, certains produits de l'invention provoquent des effets stimulants de l'acitivité motrice de la souris (SR 41678; SR 41680, SR 41881) alors que d'autres révèlent une activité sédative caractérisée par une réduction importante de la motilité des animaux (SR 41676; SR 42008; SR 42009). Le composé A n'agit pas sur la motilité spontanée.

2. Potentialisation de la narcose au pentobarbital

Dans le but d'apprécier le pouvoir hypnogéne des produits, nous avons étudié leur capacité à potentialiser les effets d'une dose subnarcotique de pentobarbital chez la souris. Les lots sont constitués de 10 souris Charles Rivers CD1, de poids compris entre 20 et 24 g.

Le pentobarbital (20 mg/kg, i.p.) est administré 60 minutes après l'administration des produits. Le critère d'endormissement retenu est la perte du réflexe de retournement. Les animaux qui ne présentent pas ce réflexe ont été dénombrés.

Tableau 3

| Produits | Potentialisation du Pentobarbital % d'animaux en narcose, produits à 100 mg/kg per os |
|---|---|
| SR 41293 | 80% DE 50= 56 (47–85) **1 |
| SR 41676 | 100% DE 50= 48 (35–66) **1 |
| SR 41677 | 30% |
| SR 41678 | 30% |
| SR 41873 | 10% |
| SR 41880 | 40% |
| SR 41881 | 30% |
| SR 41882 | 20% |
| SR 42001 | 20% |
| SR 42002 | 50% |
| SR 42004 | 30% |
| Composé A | 0% |

**1: La DE 50 a eté calculée par la méthode des probits, et l'intervalle de confiance, entre parenthèses, a été établi pour le niveau de probabilité p < ou = 0.05.

Les produits selon l'invention sont aptes à potentialiser la narcose au pentobarbital; cette propriété est prédictive d'un effet hypnogène. Le composé A est dépourvu d'effet hypnogène.

3. Evaluation de l'activité anticonvulsivante des produits

L'effet anticonvulsivant des produits chez la souris a été évalué sur un modèle de convulsions provoquées par un choc électrique, et sur 2 modèles de convulsions induites par des agents chimiques: la bicuculline et l'acide 3-mercaptopropionique.

3.a Antagonisme des convulsions induites par un choc électrique.

Le test a été légèrement modifié de celui de SWINYARD et al., J. Pharm. Exp. Ther., 1952, 106, 319–330 et ASAMI et al. Arzneim. Forsch., 1974, 24 (1), 1563–1568. Le matériel est composé d'un générateur de chocs Racia muni de 2 électrodes oculaires délivrant un courant de 60 volts pendant 0,3 seconde. Les lots sont constitués de 10 souris Charles Rivers CD1, de poids compris entre 20 et 24 g. Les produits sont administrés par voie orale, 60 minutes avant l'élcetrochoc. Les animaux ne présentant pas l'extension tonique des membres postérieurs sont considérés comme protégés de la crise convulsive.

Tableau 4

| Produits | Dose efficace médiane (DE 50) d'antagonisme de choc électrique (mg/kg per os) |
|---|---|
| SR 41293 | 99 (91–108) **1 |
| SR 41678 | 44 (36– 54) **1 |
| SR 41680 | 51 (41– 63) **1 |
| SR 41873 | 62 (48– 80) **1 |
| SR 41881 | 92 (67–128) **1 |
| SR 42002 | 49 (30– 79) **1 |

Tableau 4 (suite)

| Produits | Dose efficace médiane (DE 50) d'antagonisme de choc électrique (mg/kg per os) |
|---|---|
| SR 42004 | 34 (31– 38) **1 |
| SR 42010 | 105 (69–160) **1 |
| SR 42354 | 34 (18– 63) **1 |
| SR 42401 | 39 (22– 61) **1 |
| SR 42508 | 50 (42– 59) **1 |
| SR 42717 | 41 (31– 55) **1 |
| SR 42725 | 34 (26– 45) **1 |
| SR 42726 | 72 (58– 91) **1 |
| Composé A | DE 50 non déterminée Inhibition 40% à 200 mg/kg |

**1: La DE 50 a été calculée par la méthode des probits, et l'intervalle de confiance entre parenthèses a été établi pour le niveau de probailité p < ou = 0.05.

3.b – Antagonisme des convulsions et de la mortalité provoquées par la bicuculline.

Les lots sont constitués de 10 souris Charles Rivers CDI, de poids compris entre 20 et 22 g. Les produits sont administrés par voie orale, 60 minutes avant la bicuculline (0,8 mg/kg, i.v). L'apparition de convulsions toniques ainsi que la mortalité sont notées pendant les 60 minutes qui suivent l'injection de la bicuculline.

Tableau 5

| Produits | Dose efficace médiane (DE 50) d'antagonisme des effets de la bicuculline (mg/kg per os) Convulsions toniques | Mortalité |
|---|---|---|
| SR 41293 | 90 (33–240) **1 | 41 (30– 55) **1 |
| SR 41678 | 76 (52–111) **1 | 76 (37–155) **1 |
| SR 41873 | 112 (88–142) **1 | 85 (67–109) **1 |
| SR 42001 | 83 (36–180) **1 | 62 (53– 72) **1 |
| SR 42002 | peu différent de 100 | peu différent de 100 |
| SR 42004 | peu différent de 100 | supérieur à 100 |
| SR 42354 | 63 (34–118) **1 | 66 (31–142) **1 |
| SR 42401 | 62 (44– 87) **1 | 72 (40–129) **1 |
| SR 42508 | peu différent de 100 | 73 (33–159) **1 |
| SR 42717 | peu différent de 100 | 59 (32–109) **1 |
| SR 42725 | peu différent de 100 | 66 (40–110) **1 |
| Composé A | inactif à 200 | DE 50 non déterminée Antagonisme 30% à 200 |

**1: La DE 50 a été calculée par la méthode des probits, et l'intervalle de confiance entre parenthèses a été établi pour le niveau de probabilité p < 0,05.

3.c – Antagonisme des convulsions provoquées par l'acide 3-mercaptopropionique.

Les lots sont constitués de 10 souris Charles Rivers CD1, de poids compris entre 20 et 22 g. Les produits sont administrés par voie orale 60 minutes avant l'acide 3-mercaptopropionique (60 mg/kg s.c.). L'apparition des convulsions toniques est notée pendant les 60 minutes qui suivent l'administration d'acide 3-mercaptopropionique.

Tableau 6

| Produits | Dose efficace médiane (DE 50) d'antagonisme des convulsions provoquées par l'acide 3-mercaptopropionique (mg/kg per os) |
|---|---|
| SR 41293 | 20 (17–23) **1 |
| SR 41676 | 23 (18–29) **1 |
| SR 41678 | 53 (38–75) **1 |
| SR 41680 | 54 (36–80) **1 |
| SR 41873 | 48 (37–64) **1 |
| SR 41881 | 46 (35–62) **1 |
| SR 41882 | 41 (30–56) **1 |
| SR 42002 | 49 (32–74) **1 |
| SR 42004 | 27 (18–39) **1 |
| SR 42010 | 44 (22–90) **1 |
| SR 42354 | 40 (25–36) **1 |
| Composé A | supérieur à 100 |

**1: La DE 50 a été calculée par la méthode des probits, et l'intervalle de confiance entre parenthèses a été établi pour le niveau de probailité p < ou = à 0.05.

Après administration orale chez la souris, les produits selon l'invention manifestent des propriétés anticonvulsivantes, tant vis-à-vis du choc électrique que de la bicuculline et de l'acide 3-mercaptopropionique. Au contraire, le composé A ne présente pas de propriétés anticonvulsivantes.

La toxicité des produits selon l'invention a été étudiée: les produits sont administrés par voie orale, à des lots de 5 souris femelles Charles Rivers CD1, de poids compris entre 20 et 24 g. La toxicité est relevée pendant les 72 heures qui suivent l'administration des produits.

Tabelau 7
Determination de la dose létale chez la souris après administration aigue des produits

| Produits | P. cent de toxicité 250 mg/kg p.o. | 500 mg/kg p.o. | 1000 mg/kg per os) |
|---|---|---|---|
| SR 41293 | 0 | 0 | 80 |
| SR 41676 | 0 | 0 | 0 |
| SR 41677 | 0 | 0 | n.d. |
| SR 41678 | 0 | 0 | 60 |
| SR 41680 | 0 | 0 | n.d. |
| SR 41773 | 0 | 0 | n.d. |
| SR 41873 | 0 | 0 | n.d. |
| SR 41880 | 0 | 0 | 20 |
| SR 41881 | 0 | 0 | 100 |
| SR 41882 | 0 | 0 | 100 |
| SR 42001 | 0 | 0 | 20 |
| SR 42002 | 0 | 0 | 100 |
| SR 42003 | 0 | 0 | 0 |
| SR 42004 | 0 | 0 | 0 |
| SR 42008 | 0 | 0 | 0 |
| SR 42009 | 0 | 0 | 40 |
| SR 42010 | 0 | nd. | n.d. |
| SR 42133 | 0 | 0 | 0 |
| SR 42134 | 0 | 0 | 0 |
| SR 42354 | 0 | 0 | n.d. |
| SR 42401 | 0 | 0 | n.d. |
| SR 42508 | 0 | 0 | 0 |
| SR 42717 | 0 | 0 | 100 |
| SR 42725 | 0 | 0 | 0 |
| SR 42726 | 0 | 0 | 20 |
| Composé A | 0 | 0 | 60 |

n.d. signifie non déterminé.

Les résultats exprimés en pourcentage d'animaux qui meurent dans les 72 heures consécutives à l'adminsitration orale des produits sont notés dans le tableau précédent.

Les doses létales sont considérablement plus élevées que leurs doses actives dans les tests pharmacologiques décrits dans les précédents paragraphes.

Les essais ainsi effectués montrent que les produits selon l'invention présentent des propriétés pharmakologiques intéressantes et une faible toxicité. Par suite, ils peuvent être utilisés en thérapeutique humaine notamment pour le traitement des affections psychiques, neurologiques, ou neuromusculaires.

En particulier, les produits selon l'invention peuvent être utilisés pour le traitement des troubles de l'humeur ou du comportement: nervosisme, irritabilité ainsi que pour les traitements des états anxieux, des insomnies et de l'épilepsie.

Ces produits peuvent être administrés par voie orale ou par voie injectable. Les compositions pharmaceutiques peuvent être solides ou liquides et se présenter, par exemple, sous forme de comprimés, gélules, granulés, suppositoires ou préparations injectables.

La posologie peut varier dans de larges proportions, en particulier suivant le type et la gravité de l'affection à traiter et suivant le mode d'administration. Le plus souvent, chez l'adulte par voie orale, elle est comprise entre 1 mg et 500 mg par jour, éventuellement répartie en plusieurs prises.

A titre d'exemple, on peut indiquer la préparation galénique suivante:

| Gelules: | |
|---|---|
| SR 42354 | 50 mg |
| Aérosil | 0,5 mg |
| Stéarate de magnésium | 1,5 mg |
| Amidon STA RX 1500 | 48 mg |
| | 100 mg |

**Revendications pour les états contractants BE, CH, DE, FR, GB, IT, LI, LU, NL, SE**

1. Pyrrolidinones de formule (I):

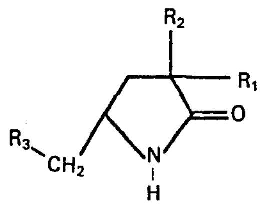

(I)

dans laquelle:

$R_1$ représente un atome d'hydrogène ou un groupe alkyle droit ou ramifié ayant 1 à 4 atomes de carbone;

$R_2$ représente un groupe alkyle ayant 1 à 4 atomes de carbone, un groupe phényle ou un groupe triméthylsilyle;

$R_3$ représente un groupe thiényl-2 ou un groupe phényle éventuellement substitué 1 ou 2 fois par un atome d'halogène, un groupe méthyle, un groupe méthoxy ou un groupe trifluorométhyle.

2. Composés selon la revendication 1, caractérisés en ce que $R_3$ représente un groupe chlorophényle ou trifluorométhylphényle.

3. Composé selon la revendication 1, caractérisé en ce que $R_2$ représente un groupe méthyle, $R_1$ représente l'hydrogène et $R_3$ représente un groupe chloro-4 phényle.

4. Composé selon la revendication 1, caractérisé en ce que $R_2$ représente un groupe méthyle, $R_1$ représente l'hydrogène et $R_3$ représente un groupe trifluorométhyl-3 phényle.

5. Composés selon la revendication 1, caractérisés en ce que $R_2$ représente un groupe méthyle, $R_1$ représente l'hydrogène et $R_3$ représente un groupe chloro-2 phényle ou chloro-3 phényle.

6. Composé selon la revendication 1, caractérisé en ce que $R_1$ et $R_2$ représentent chacun un groupe méthyle et $R_3$ représente un groupe trifluorométhyl-3 phényle.

7. Procédé de préparation de produits de formule (I), caractérisé en ce que l'on effectue la cyclisation d'un ester γ-aminé de formule (II):

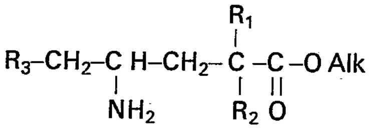

(II)

dans laquelle $R_1$, $R_2$, $R_3$ sont tels que définis dans la revendication 1, Alk représente un groupe alkyle.

8. Procédé de préparation de produits de formule (I) mono u disubstitués en position 3 par un groupement alkyle ou triméthylsilyle, caractérisé en ce qu'il consiste:

– à utiliser, comme produit de départ, le composé de formule:

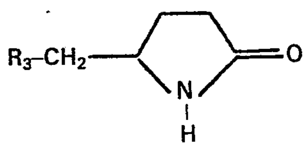

et à protéger le groupe NH à l'aide d'un groupement triméthylsilyle;

– à faire réagir le produit ainsi obtenu avec le lithium diisopropylamide puis avec un halogénure $R_2$ Z dans lequel Z est l'halogène de façon à obtenir le mélange des produits:

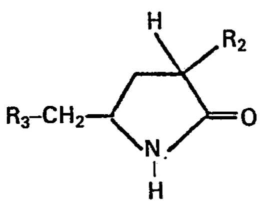 et 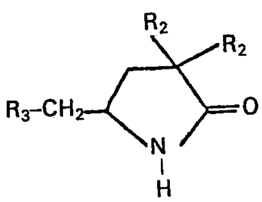

(I') (I'')

qu'on sépare par chromatographie;

– éventuellement, à soumettre le composé de formule (I') aux opérations précédentes avec un halogénure $R_1$ Z pour préparer un composé de formule (I) dans lequel $R_1$ et $R_2$ sont différents.

9. Composition pharmaceutiques pour le traitement des affections psychiques, neurologiques ou neuromusculaires, caractérisées en ce qu'elles contiennent au moins un composé selon la revendication 1, en association avec un véhicule pharmaceutiquement acceptable.

10. Compositions pharmaceutiques selon la revendication 9, caractérisées en ce qu'elles contiennent 1 à 500 mg d'un composé selon la revendication 1 et sont conditionnées pour l'administration par voie orale ou par voie injectable.

**Revendications pour l'état contractant AT**

1. Procédé pour l'obtention de pyrrolidinones de formule (I):

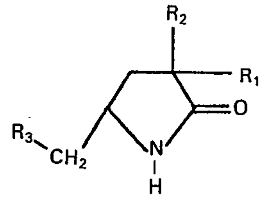

(I)

dans laquelle:

– $R_1$ représente un atome d'hydrogène ou un groupe alkyle droit ou ramifié ayant 1 à 4 atomes de carbone,

– $R_2$ représente un groupe alkyle ayant 1 à 4 atomes de carbone, un groupe phényle ou un groupe triméthylsilyle

– $R_3$ représente un groupe thiényl-2 ou un groupe phényle éventuellement substitué 1 ou 2 fois par un atome d'halogène, un groupe méthyle, un groupe méthoxy ou un groupe trifluorométhyle, caractérisé en ce qu'il consiste à effectuer la cyclisation d'un ester γ-aminé de formule (II):

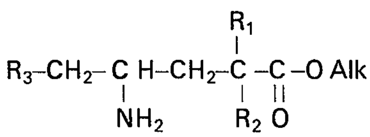

(II)

dans laquelle $R_1$, $R_2$ et $R_3$ sont tels que définis cidessus et Alk est un groupe alkyle.

2. Procédé pour l'obtention de pyrrolidinones de formule (I):

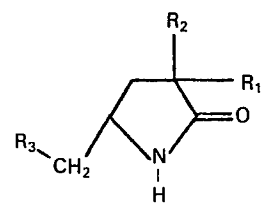

(I)

dans laquelle:

– $R_1$ représente un atome d'hydrogène ou un groupe alkyle droit ou ramifié ayant 1 à 4 atomes de carbone

– $R_2$ représente un groupe alkyle ayant 1 à 4 atomes de carbone, un groupe triméthylsilyle

– $R_3$ représente un groupe thiényl-2 ou un groupe phényle éventuellement substitué 1 ou 2 fois par un atome d'halogène, un groupe méthyle, un groupe méthoxy ou un groupe trifluorométhyle, caractérisé en ce qu'il consiste:

– à utiliser, comme produit de départ, le composé de formule:

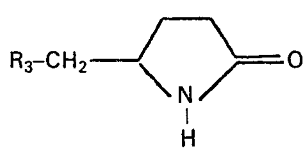

et à protéger le groupe NH à l'aide d'un groupement triméthylsilyle;

– à faire réagir le produit ainsi obtenu avec le lithium diisopropylamide puis avec un halogénure $R_2$ Z dans lequel Z est l'halogène de façon obtenir le mélange des produits:

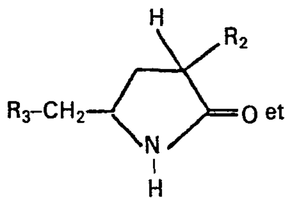 et 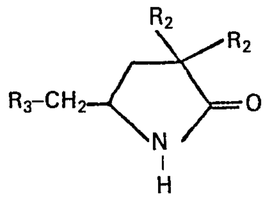

(I') (I'')

qu'on sépare par chromatographie;

– éventuellement, à soumettre le composé de formule (I') aux opérations précédentes avec un halogénure $R_1$ Z pour préparer un composé de formule (I) dans lequel $R_1$ et $R_2$ sont différents.

3. Procédé selon l'une des revendications 1 ou 2 pour la préparation de pyrrolidinones de formule (I) dans laquelle $R_3$ représente un groupe chlorophényle ou trifluorométhylphényle.

4. Procédé selon l'une des revendications 1 ou 2 pour la préparation de la pyrrolidinone de formule (I) dans laquelle $R_2$ représente un groupe méthyle, $R_1$ représente l'hydrogène et $R_3$ représente un groupe chloro-4 phényle.

5. Procédé selon l'une des revendications 1 ou 2, pour la préparation de la pyrrolidinone de formule (I) dans laquelle $R_2$ représente un groupe méthyle, $R_1$ représente l'hydrogène et $R_3$ représente un groupe trifluorométhyl-3 phényle.

6. Procédé selon l'une des revendications 1 ou 2 pour la préparation des pyrrolidinones de formule (I) dans laquelle $R_2$ représente un groupe méthyle, $R_1$ représente l'hydrogène et $R_3$ représente un groupe chloro-2 phényle ou chloro-3 phényle.

7. Procédé selon l'une des revendications 1 ou 2 pour la préparation de la pyrrolidinone de formule (I) dans laquelle $R_1$ et $R_2$ représentent chacun un groupe méthyle et $R_3$ représente un groupe trifluorométhyle-3 phényle.

8. Utilisation des pyrrolidinones de formule (I) obtenues selon l'une quelconque des revendications 1 à 7, en association avec un véhicule pharmaceutiquement acceptable, pour la préparation de compositions pharmaceutiques pour le traitement des affections psychiques.

9. Utilisation selon la revendication 8 pour la préparation de compositions pharmaceutiques contenant de 1 à 500 mg d'une pyrrolidinone de formule (I), conditionnées pour l'administration par voie orale ou par voie injectable.

**Patentansprüche für die Vertragsstaaten BE, CH, DE, FR, GB, IT, LI, LU, NL, SE:**

1. Pyrrolidinone der Formel (I):

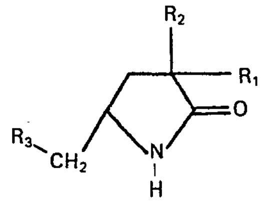 (I)

worin:

$R_1$ ein Wasserstoffatom oder eine gerade oder verzweigte Alkylgruppe mit 1 bis 4 Kohlenstoffatomen darstellt,

$R_2$ eine Alkylgruppe mit 1 bis 4 Kohlenstoffatomen, eine Phenylgruppe oder eine Trimethylsilylgruppe darstellt,

$R_3$ eine 2-Thienylgruppe oder eine Phenylgruppe, gegebenenfalls 1- oder 2mal substituiert durch ein Halogenatom, eine Methylgruppe, eine Methoxygruppe oder eine Trifluormethylgruppe darstellt.

2. Verbindungen nach Anspruch 1, dadurch gekennzeichnet, dass $R_3$ eine Chlorphenyl- oder Trifluormethylphenylgruppe darstellt.

3. Verbindungen nach Anspruch 1, dadurch gekennzeichnet, dass $R_2$ eine Methylgruppe, $R_1$ Wasserstoff und $R_3$ eine 4-Chlorphenylgruppe darstellt.

4. Verbindungen nach Anspruch 1, dadurch gekennzeichnet, dass $R_2$ eine Methylgruppe, $R_1$ Wasserstoff und $R_3$ eine 3-Trifluormethylphenylgruppe darstellt.

5. Verbindungen nach Anspruch 1, dadurch gekennzeichnet, dass $R_2$ eine Methylgruppe, $R_1$ Wasserstoff und $R_3$ eine 2-Chlorphenyl- oder 3-Chlorphenylgruppe darstellt.

6. Verbindungen nach Anspruch 1, dadurch gekennzeichnet, dass $R_1$ und $R_2$ jeweils eine Methylgruppe und $R_3$ eine 3-Trifluormethylgruppe darstellen.

7. Verfahren zur Herstellung von Produkten der Formel (I), dadurch gekennzeichnet, dass man die Zyklisierung eines γ-aminierten Esters der Formel (II):

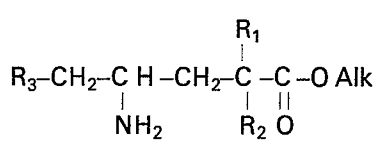 (II)

vornimmt, worin $R_1$, $R_2$, $R_3$ wie in Anspruch 1 definiert sind und Alk eine Alkylgruppe bedeutet.

8. Verfahren zur Herstellung von Produkten der Formel (I), die in der Position 3 durch eine Alkyl-oder Trimethylsilylgruppe mono- oder disubstituiert sind, dadurch gekennzeichnet, dass

– als Ausgangsprodukt die Verbindung der Formel::

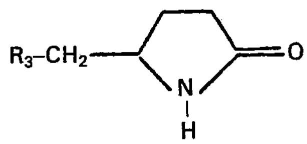

verwendet wird und die Gruppe NH mit Hilfe einer Trimethylsilylgruppe geschützt wird:

– das so erhaltene Produkt mit Lithiumdiisopropylamid und dann mit einem Halogenid $R_2Z$, worin Z für Halogen steht, derart reagieren gelassen wird, dass eine Mischung der Produkte:

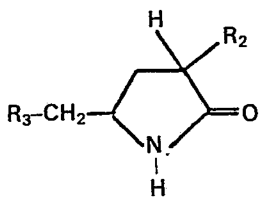 und 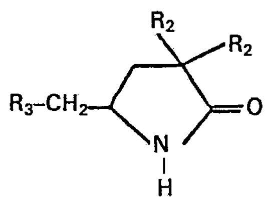

(I′) (I″)

erhalten wird, die man mittels Chromatographie trennt:

– gegebenenfalls die Verbindung der Formel (I′) den vorherigen Vorgängen mit einem Halogenid $R_1Z$ zur Herstellung einer Verbindung der Formel (I), worin $R_1$ und $R_2$ unterschiedlich sind, unterzieht.

9. Pharmazeutische Zusammensetzungen zur Behandlung von psychischen, neurologischen und neuromuskulären Erkrankungen, dadurch gekennzeichnet, dass sie mindestens eine Verbindung nach Anspruch 1 in Verbindung mit einem pharmazeutisch akzeptablen Träger enthalten.

10. Pharmazeutische Zusammensetzungen nach Anspruch 9, dadurch gekennzeichnet, dass sie 1 bis 500 mg einer Verbindung nach Anspruch 1 enthalten und zur Verabreichung auf oralem Weg oder auf Injektionsweg konditioniert sind.

**Patentansprüche für den Vertragsstaat AT:**

1. Verfahren zur Herstellung von Pyrrolidononen der Formel (I):

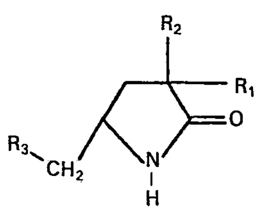

(I)

worin:

$R_1$ ein Wasserstoffatom oder eine gerade oder verzweigte Alkylgruppe mit 1 bis 4 Kohlenstoffatomen darstellt,

$R_2$ eine Alkylgruppe mit 1 bis 4 Kohlenstoffatomen, eine Phenylgruppe oder eine Trimethylsilylgruppe darstellt,

$R_3$ eine 2-Thienylgruppe oder eine Phenylgruppe, gegebenenfalls 1- oder 2mal substituiert durch ein Halogenatom, eine Methylgruppe, eine Methoxygruppe oder eine Trifluormethylgruppe darstellt, dadurch gekennzeichnet, dass man die Zyklisierung eines γ-aminierten Esters der Formel (II):

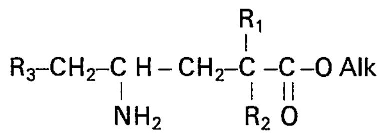

(II)

vornimmt, worin $R_1$, $R_2$, $R_3$ wie oben definiert sind und Alk eine Alkylgruppe bedeutet.

2. Verfahren zur Herstellung von Pyrrolidinonen der Formel (I):

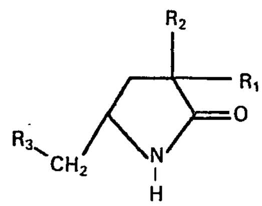

(I)

worin:

$R_1$ ein Wasserstoffatom oder eine gerade oder verzweigte Alkylgruppe mit 1 bis 4 Kohlenstoffatomen darstellt,

$R_2$ eine Alkylgruppe mit 1 bis 4 Kohlenstoffatomen oder eine Trimethylsilylgruppe darstellt,

$R_3$ eine 2-Thienylgruppe oder eine Phenylgruppe, gegebenenfalls 1- oder 2mal substituiert durch ein Halogenatom, eine Methylgruppe, eine Methoxygruppe oder eine Trifluormethylgruppe darstellt, dadurch gekennzeichnet, dass man

– als Ausgangsprodukt die Verbindung der Formel:

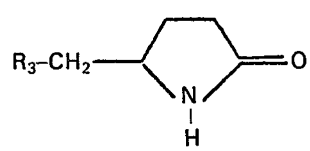

verwendet und die Gruppe NH mit Hilfe einer Trimethylsilylgruppe geschützt wird;

– das so erhaltene Produkt mit Lithiumdiisopropylamid und dann mit einem Halogenid $R_2Z$, worin Z für Halogenid steht, derart reagieren gelassen wird, dass eine Mischung der Produkte:

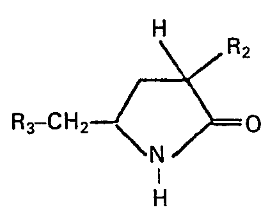 und 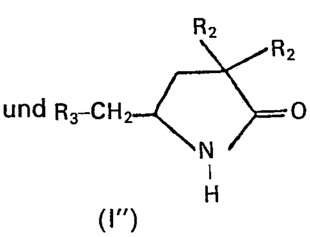

(I′) (I″)

erhalten wird, die man mittels Chromatographie trennt;

– gegebenenfalls die Verbindung der Formel (I') den vorherigen Vorgängen mit einem Halogenid $R_1Z$ zur Herstellung einer Verbindung der Formel (I), worin $R_1$ und $R_2$ unterschiedlich sind, unterzieht.

3. Verfahren nach einem der Ansprüche 1 oder 2 zur Herstellung von Pyrrolidinonen der Formel (I), worin $R_3$ eine Chlorphenyl- oder Trifluormethylphenylgruppe darstellt.

4. Verfahren nach einem der Ansprüche 1 oder 2 zur Herstellung des Pyrrolidinons der Formel (I), worin $R_2$ eine Methylgruppe, $R_1$ Wasserstoff und $R_3$ eine 4-Chlorphenylgruppe darstellt.

5. Verfahren nach einem der Ansprüche 1 oder 2 zur Herstellung des Pyrrolidinons der Formel (I), worin $R_2$ eine Methylgruppe, $R_1$ Wasserstoff und $R_3$ eine 3-Trifluormethylphenylgruppe darstellt.

6. Verfahren nach einem der Ansprüche 1 oder 2 zur Herstellung der Pyrrolidinone der Formel (I), worin $R_2$ eine Methylgruppe, $R_1$ Wasserstoff und $R_3$ eine 2-Chlorphenyl- oder 3-Chlorphenylgruppe darstellt.

7. Verfahren nach einem der Ansprüche 1 oder 2 zur Herstellung des Pyrrolidinons der Formel (I), worin $R_1$ und $R_2$ jeweils eine Methylgruppe und $R_3$ eine 3-Trifluormethylphenylgruppe darstellt.

8. Verwendung der nach einem der Ansprüche 1 bis 7 erhaltenen Pyrrolidinone der Formel (I) in Verbindung mit einem pharmazeutisch akzeptablen Träger zur Herstellung von pharmazeutischen Zusammensetzungen zur Behandlung von psychischen Erkrankungen.

9. Verwendung nach Anspruch 8 zur Herstellung von pharmazeutischen Zusammensetzungen, die 1 bis 500 mg eines Pyrrolidinons der Formel (I) enthalten und zur Verabreichung auf oralem Weg oder auf Injektionsweg konditioniert sind.

**Claims for the contracting states: BE, CH, DE, FR, GB, IT, LI, LU, NL, S**

1. Pyrrolidinones of the formula (I):

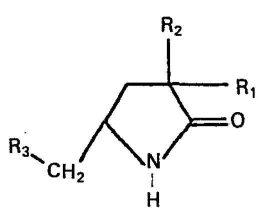

(I)

in which:

– $R_1$ represents a hydrogen atom or a straight or branched alkyl group having 1 to 4 carbon atoms;

– $R_2$ represents an alkyl group having 1 to 4 carbon atoms, a phenyl group or a trimethylsilyl group;

– $R_3$ represents a 2-thienyl group or a phenyl group possibly substitued once or twice with a halogen atom, a methyl group, a methoxy group or a trifluoromethyl group,

2. Compounds according to claim 1, characterized in that $R_3$ represents a chlorophenyl or a trifluoromethylphenyl group.

3. Compound according to claim 1, characterized in that $R_2$ represents a methyl group, $R_1$ represents hydrogen and $R_3$ represents a 4-chloro-phenyl group.

4. Compound according to claim 1, characterized in that $R_2$ represents a methyl group, $R_1$ represents hydrogen and $R_3$ represents a 3-trifluoromethyl -phenyl group.

5. Compounds according to claim 1, characterized in that $R_2$ represents a methyl group, $R_1$ represents hydrogen and $R_3$ represents 2-chloro-phenyl or 3-chloro-phenyl group.

6. Compound according to claim 1, characterized in that $R_1$ and $R_2$ each represent a methyl group and $R_3$ represents a 3-trifluoromethyl phenyl group.

7. Process for the preparation of products of Formula (I), characterized in that it comprises the cyclization of a gamma-amino ester of Formula (II):

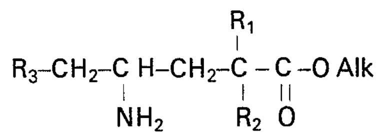

(II)

in which $R_1$, $R_2$ and $R_3$ are as defined in claim 1, and Alk represents an alkyl group.

8. Process for the preparation of products of the formule (I) mono or disubstituted at the 3 position by an alkyl or trimethylsilyl group, characterized in that it consists in using as starting material, the compound of formula:

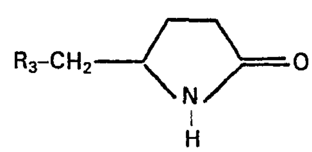

and protecting the NH group by means of a trimethylsilyl group;

– reacting the product thus obtained with lithium diisopropylamide and then with halide $R_2Z$ in which Z is the halogen so as to obtain the mixture of products.

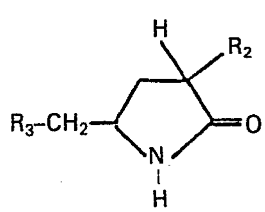

and

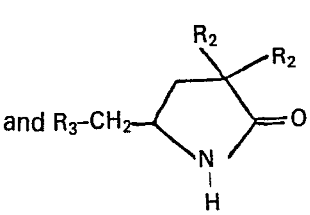

(I') (I'')

separating said mixture by chromatography;

– and optionally subjecting the compound of formula (I') to the foregoing operations with a halide $R_1$ Z to prepare a compound of formula (I) in which $R_1$ and $R_2$ are different.

9. Pharmaceutical compositions for treatment of psychic, neurological or neuromuscular disorders, characterized in that they contain at least one compound according to claim 1, in association with a pharmaceutically acceptable vehicle.

10. Pharmaceutical compositions according to claim 9, characterized in that they comprise 1 to 500 mg of a compound according to claim 1 and are packaged for oral or injectable administration.

**Claims for the contracting state: AT**

1. Process for the production of pyrrolidinones of the formula (I):

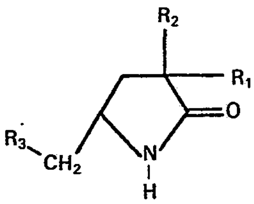

(I)

in which – $R_1$ represents a hydrogen atom or a straight or branched alkyl group having 1 to 4 carbon atoms;

– $R_2$ represents an alkyl group having 1 to 4 carbon atoms, a phenyl group or a trimethylsilyl group;

– $R_3$ represents a 2-thienyl group or a phenyl group optionally substituted once or twice by a halogen atom, a methyl group, a methoxy group or a trifluoromethyl group; characterized in that consists in carrying out the cyclization of a gamma-amino ester of the formule (II):

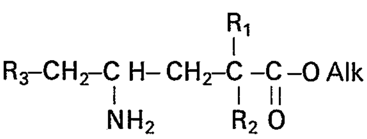

(II)

in which $R_1$, $R_2$ and $R_3$ are as defined above and Alk is an alkyl group.

2. Process for the production of pyrrolidinones of the formula (I)

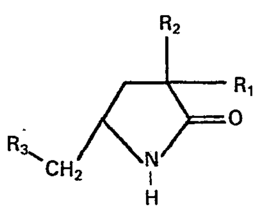

(I)

in which:

– $R_1$ represents a hydrogen atom or a straight or branched alkyl group having 1 to 4 carbon atoms;

– $R_2$ represents an alkyl group having 1 to 4 carbon atoms, a trimethylsilyl group;

– $R_3$ represents a 2-thienyl group or a phenyl group optionally substituted once or twice by a halogen atom, a methyl group, a methoxy group or a trifluoromethyl group; characterized in that it consists in using as starting material, the compound of formula:

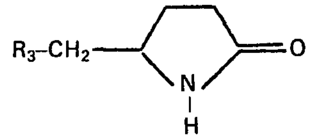

and protecting the NH group by means of a trimethylsilyl group;

– reacting the product so obtained with lithium diisopropylamide and then with a halide $R_2$ Z in which Z is the halogen so as to obtain the mixture of products:

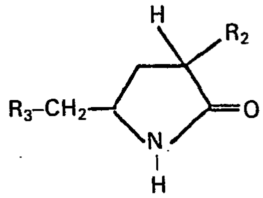

and

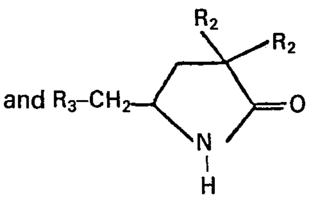

(I') (I'')

separating these by chromatography;

– and optionally subjecting the compound of formula (I') to the foregoing operations with a halide $R_1$ Z to prepare a compound of formule (I) in which $R_1$ and $R_2$ are different.

3. Process according to any one of claims 1 or 2, for the production of pyrrolidinones of formule (I) in which $R_3$ represents a chlorophenyl or a trifluoromethylphenyl group.

4. Process according to any one of claims 1 or 2, for the production of pyrrolidinones of formula (I) in which $R_2$ represents a methyl group, $R_1$ represents hydrogen and $R_3$ represents a 4-chloro-phenyl group.

5. Process according to any one of claims 1 or 2, for the production of pyrrolidinones of formula (I) in which $R_2$ represents a methyl group, $R_1$ represents hydrogen and $R_3$ represents a 3-trifluoromethyl-phenyl group.

6. Process according to any one of claims 1 or 2, for the production of pyrrolidinones of formula (I) in which $R_2$ represents a methyl group, $R_1$ represents hydrogen and $R_3$ represents 2-chlorophenyl or 3-chlorophenyl group.

7. Process according to any one of claims 1 or 2, for the production of pyrrolidinones of formula (I) in which $R_1$ and $R_2$ each represent a methyl group and $R_3$ represents a 3-trifluoromethyl-phenyl group.

8. Use of pyrrolidinones of formula (I) obtained according to any one of claims 1 to 7, in association with a pharmaceutically acceptable vehicle, for the preparation of pharmaceutical compositions for the treatment of psychic disorders.

9. Use according to claim 8, for the preparation of pharmaceutical compositions containing 1 to 500 mg of a pyrrolidinone of formula (I), packaged for oral or injectable administration.